# EUROPEAN PATENT APPLICATION

(11) **EP 2 182 075 A1**
(43) Date of publication of application: **05.05.2010**
(21) Application number: 08167031.7
(22) Date of filing: 20.10.2008
(51) Int. Cl.: C12Q 1/68

(54) **Real-time high multiplex detection by primer extension on solid surfaces**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention provides a method that allows for real-time detection of a multitude of target nucleic acids of interest in one reaction (multiplexing) using detectable labels of a single color. The method comprises directly amplifying one or more target nucleic acids on a solid substrate thus providing an enhanced signal to noise ratio and reduced assay times. Further provided is a diagnostic kit for monitoring the amplification of one or more target nucleic acids.

## Description

### FIELD OF THE INVENTION

The present invention provides a method that allows for real-time detection of a multitude of target nucleic acids of interest in one reaction (multiplexing) using detectable labels of a single color. The method comprises directly amplifying one or more target nucleic acids on a solid substrate, thus providing an enhanced signal to noise ratio and reduced assay times. Further provided is a diagnostic kit for monitoring the amplification of one or more target nucleic acids.

### BACKGROUND OF THE INVENTION

Techniques for the detection and amplification of extremely small quantities of nucleic acid are an indispensable tool in modem molecular biology and biochemical research and are e.g. used for the diagnosis and detection of diseases, in forensic science, DNA sequencing and recombinant DNA technology.

The use of the polymerase chain reaction (PCR) offers a fast and convenient method of amplifying a specific nucleic acid sequence. The technique is based on the replication of nucleic acids using a thermostable DNA-Polymerase.

A basic PCR setup requires several components and reagents. A denatured nucleic acid sample is incubated with DNA polymerase, nucleotides and two oligonucleotide primers, which are chosen such that they flank the fragment to be amplified so that they direct the DNA polymerase to synthesize new complementary strands.

PCR methods commonly involve thermal cycling, i.e., alternately heating and cooling the PCR sample to a defined series of temperature steps. Most commonly PCR is carried out with 20-40 cycles each having 3 different temperature steps. In a first step the reaction is heated (e.g. to 94-98°C) in order to melt the nucleic acid template by disrupting the hydrogen bonds between complementary bases of the nucleic acid strands (denaturation step). Next the reaction temperature is lowered to a temperature that corresponds to the melting temperature of the primers used (e.g. 50° to 65° C) in order to allow annealing of the primers to their complementary sequences on the single stranded nucleic acid template (annealing step). In the third step the DNA polymerase synthesizes a new nucleic acid strand by adding nucleotides that are complementary to the template in 5' to 3' direction (elongation step; e.g. carried out at 72° C). As PCR progresses, the nucleic acid thus generated is itself used as a template for replication. This causes a chain reaction in which the nucleic acid template is exponentially amplified. Approximately 20 cycles of PCR amplification increase the amount of the target sequence around one-millionfold with high specificity. However, this PCR method is at best semi-quantitative and, in many cases, the amount of product is not related to the amount of input target nucleic acid.

For some applications, for example diagnostic methods or gene expression studies, it is however desirable to monitor the increase in the amount of nucleic acid as it is amplified. This can be achieved by a quantitative PCR method that has been introduced fairly recently and which is referred to as "real-time PCR". The procedure follows the general principle of polymerase chain reaction, with the amplified nucleic acid being quantified in real time as it accumulates in the reaction at the respective PCR cycles. The quantification is usually based on fluorescent measurements. Most commonly an intercalating dye (e.g. SYBR green) which binds to double stranded nucleic acid molecules in the PCR reaction is used to give fluorescence. An increase in nucleic acid product during PCR thus leads to an increase in fluorescence intensity and is measured at a given number of cycles or at each cycle, thus allowing nucleic acid concentrations to be quantified.

Concentrations of nucleic acid present during the exponential phase of the PCR reaction can e.g. be detected by plotting fluorescence against cycle number on a logarithmic scale. Amounts of nucleic acid can then be determined by comparing the results to a standard curve produced by real time PCR of serial dilutions of a known amount of nucleic acid. Relative concentrations of nucleic acid present during the exponential phase can e.g. also be calculated by determining a threshold for detection of fluorescence above background and calculating relative amounts of nucleic acid based on the cycle threshold of the sample.

Typically the above described real time PCR method is carried out in solution.

One major disadvantage of conventional real time PCR is that it cannot be easily applied to the detection of multiple nucleic acids in parallel (multiplexing), as different non-overlapping fluorescent dyes have to be used for different target nucleic acids. In cases where intercalating dyes are used for detection, multiplexing is not applicable at all.

Therefore, there is a need for a simple, time and cost efficient method by which different target nucleic acids can be simultaneously amplified and detected in real time within the same system. It is further desirable that such a method would provide a high signal-to-noise ratio.

### SUMMARY OF THE INVENTION

It is therefore an object of the present invention to provide a simple and efficient method for simultaneously monitoring the amplification of one or more target nucleic acids.

This and other objectives as they will become apparent from the ensuing description and claims are attained by the subject matter of the independent claims. Some of the preferred embodiments are defined by the dependent claims.

In a first aspect, the present invention relates to a method for monitoring the amplification of one or more target nucleic acids comprising the following steps:
a) providing a substrate having immobilized upon its surface a multitude of oligonucleotide primers;
b) adding to said substrate a sample of one or more target nucleic acids and further reagents required for nucleic acid amplification and labeling in a polymerase chain reaction process;
c) amplifying the one or more target nucleic acids by a process involving thermocycling, comprising the steps of:
   i) annealing the one or more target nucleic acids to corresponding oligonucleotide primers immobilized on the surface; and
   ii) extension of the immobilized oligonucleotide primers, wherein the immobilized oligonucleotide primers get detectably labeled during extension;
d) measuring the signal of the extended and labeled oligonucleotide primers during or after at least one thermal cycle.

In a preferred embodiment, the multitude of oligonucleotide primers are capable of specifically binding to a plurality of different target nucleic acids.

In a further preferred embodiment the multitude of oligonucleotide primers are located to separate regions on the surface of the substrate.

In another preferred embodiment the oligonucleotide primers within each individual region are capable of specifically binding to the same or similar target nucleic acids.

In yet another preferred embodiment some or all regions on the substrate differ from each other in that their oligonucleotide primers are capable of specifically binding to different target nucleic acids.

In still another preferred embodiment the immobilized oligonucleotide primers get labeled with a fluorescent label during the extension in step c).

In yet another preferred embodiment the fluorescent label is a fluorescently labeled nucleotide.

In a further preferred embodiment the oligonucleotide primers immobilized upon the substrate in step a) have a length of about 5 to 100 nucleotides, preferably of about 10 to 50 nucleotides, most preferably of about 15 to 30 nucleotides.

In another preferred embodiment the oligonucleotide primers specifically bind to the 5' or 3' end of an open reading frame of a target nucleic acid.

In another preferred embodiment in step d) only labels are measured which are located within a distance of about 100 nm to about 300 nm from the surface of the substrate when using an evanescent detection scheme or in step d) only labels are measured which are located within a distance of about 1 µm or less when using a confocal detection scheme.

In a further preferred embodiment the fluorescent signal of the extended labeled oligonucleotide primers is measured in step d) during or after at least 2 thermal cycles, during or after at least 5 thermal cycles, during or after at least 10 thermal cycles, during or after least 15 thermal cycles, during or after at least 20 thermal cycles or during or after at least 25 thermal cycles.

In another preferred embodiment the reagents required for nucleic acid amplification and labeling comprise forward and/or reverse primers specific for said one or more target nucleic acids.

In yet another preferred embodiment the thermocycling in c) comprises about 5 to 50 thermocyles.

In another aspect the present invention relates to the use of a method according to the invention for detecting in a sample the presence, absence or the amount of one or more target nucleic acids.

In a further aspect a diagnostic kit is provided for monitoring the amplification of one or more target nucleic acids, comprising:
(i) a substrate having immobilized upon its surface a multitude of oligonucleotide primers; and
(ii) further reagents required for nucleic acid amplification and labeling in a polymerase chain reaction process;
wherein the reagents required for nucleic acid amplification and labeling comprise nucleotides labeled with a fluorescent dye.

### BRIEF DESCRIPTION OF THE DRAWINGS

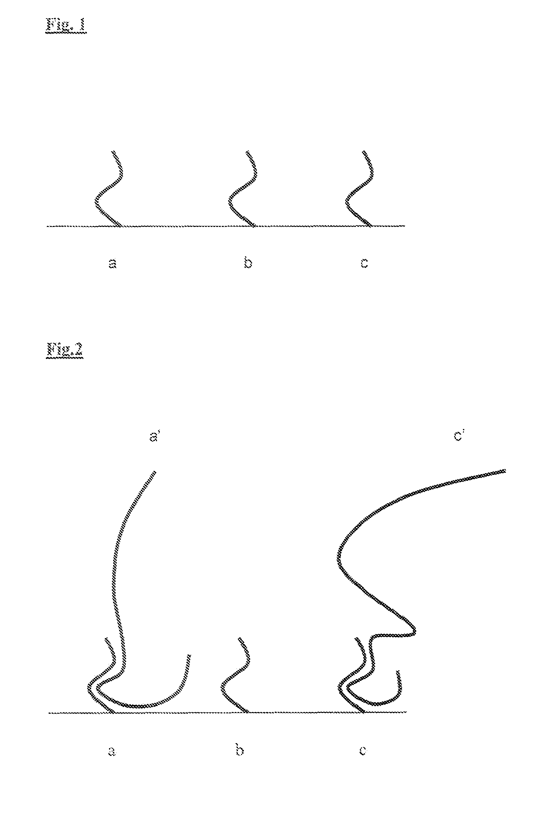

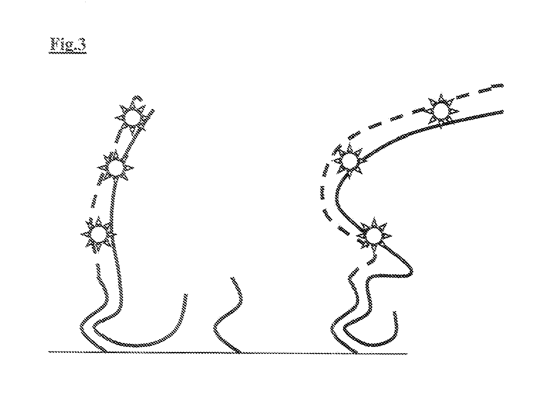

Fig.1 Scheme showing oligonucleotide primers for three different target nucleic acids being immobilized on a substrate. The primers being specific for different target nucleic acids are indicated by a, b and c in figures 1 to 3.
Fig. 2 Scheme showing hybridization of target nucleic acids present in solution onto the oligonucleotide primers during annealing in step c) of the method of the invention. In the example shown, only target nucleic acids complementary to oligonucleotide primer a and c are present (designated as a' and c', respectively).
Fig.3 Scheme showing extension and labeling of the oligonucleotide primers complementary to the targets. Extended and labeled oligonucleotide primers can subsequently be detected, even after denaturing the double strands at higher temperatures. Newly synthesized strands are depicted as dashed lines. The incorporated labeled nucleotides according to a preferred embodiment of the method of the invention are depicted as stars.
Fig. 4-Fig. 4 (a) shows fluorescent background signal as a function of time.
Fig. 4 (b) shows fluorescent background signal as a function of temperature.

### DETAILED DESCRIPTION OF EMBODIMENTS

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

The following definitions are introduced:
As used in this specification and in the intended claims, the singular forms of "a" and "an" also include the respective plurals unless the context clearly dictates otherwise.

It is to be understood that the term "comprise", and variations such as "comprises" and "comprising" is not limiting. For the purpose of the present invention the term "consisting of" is considered to be a preferred embodiment of the term "comprising". If hereinafter a group is defined to comprise at least a certain number of embodiments, this is meant to also encompass a group which preferably consists of these embodiments only.

The terms "nucleic acid" or "nucleic acid molecule" refer to a deoxyribonucleotide or ribonucleotide polymer in either single-or double-stranded form, and also encompass known analogues of natural nucleotides that can function in a similar manner as naturally occurring nucleotides.

The term "label" as used herein means a molecule or moiety having a property or characteristic which is capable of detection. Examples of labels are fluorophores, chemiluminophores, fluorescent microparticles and the like.

The term "target nucleic acid" refers to a nucleic acid, often derived from a biological sample, to which the oligonucleotide primers on the substrate can specifically hybridize or can potentially hybridize. It is recognized that the target nucleic acids can be derived from essentially any source of nucleic acids (e.g., including, but not limited to chemical syntheses, amplification reactions, forensic samples, etc.) The presence or absence of one or more target nucleic acids may be detected, or the amount of one or more target nucleic acids may be quantified by the methods disclosed herein. Target nucleic acid(s) preferentially have nucleotide sequences that are complementary to the nucleic acid sequences of the corresponding oligonucleotide primers to which they can specifically bind. However, with regard to cases where the presence or absence of one or more target nucleic is to be detected, the term "target nucleic" acids may also refer to nucleic acids present in a query sample that might potentially hybridize to the oligonucleotides on the substrate.

A target nucleic acid may e.g. be a gene, DNA, cDNA, RNA, mRNA or fragments thereof. A target nucleic acid is preferably longer than an "oligonucleotide primer" as described below and used in the context of the method of the invention.

The term "oligonucleotide primer" or "primer" as used herein refers to a specific oligonucleotide sequence which is capable of hybridizing to a target nucleotide sequence. Preferably, a "primer" is complementary to the target nucleotide sequence. A primer typically serves as an initiation point for nucleotide polymerization catalyzed by an enzyme such as, for example, a DNA polymerase, RNA polymerase or reverse transcriptase. Primers in one embodiment are **characterized in that** at least the 3 bases closest to their 3'end are complementary to the corresponding bases of the target sequence.

The term "multiplexing" as used herein refers to a process that allows for simultaneous amplification of many target nucleic acids of interest in one reaction by using more than one pair of primers. For example, said process might be Multiplex PCR. The terms "background", "background signal" or "background fluorescence" refer to signals resulting from non-specific binding, or other interactions, between labeled target nucleic acids, labeled nucleotides, labeled oligonucleotide primers or any other labeled components, such as auto-fluorescent molecules. Background signals may e.g. also be produced by intrinsic fluorescence of the substrate or its components themselves or by any unbound labeled molecules being present in the solution on top of the substrate.

The term "amplicon" or "amplicons", as used herein, refers the products of the amplification of nucleic acids, using e.g. PCR or any other method suitable for the amplification of nucleic acids. In one embodiment, the length of an amplicon is between 100 and 800 bases, preferably between 100 and 400 bases and more preferably between 100 and 200 bases.

If an oligonucleotide primer or any other nucleic acid molecule described herein is said to be "specific" for a target nucleic acid or any other nucleotide sequence or to "specifically" bind to a target nucleic acid or any other nucleotide sequence this refers to preferential binding, duplexing, or hybridizing of said oligonucleotide primer or any other nucleic acid molecule to a particular nucleotide sequence under stringent conditions. The term "stringent conditions" refers to conditions under which a probe will hybridize preferentially to its target sequence, and to a lesser extent to, or not at all to, other sequences. Stringent conditions are sequence-dependent and will be different in different circumstances.

Stringent conditions in this context may for example be selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. For example, stringent conditions may be those in which the salt concentration is at least about 0.01 to 1.0 M Na ion concentration (or other salts) at pH 7.0 to 8.3 and the temperature is at least about 30°C for short probes (e.g., 10 to 50 nucleotides). Stringent conditions may also be achieved with the addition of destabilizing agents such as formamide.

The term "quantifying" as used herein with regard to nucleic acid abundances or concentrations may refer to absolute or to relative quantification. Absolute quantification may e.g. be accomplished by inclusion of known concentration(s) of one or more target nucleic acids (control nucleic acids) and referencing the signal intensity of target nucleic acids of unknown concentration with the target nucleic acids of known concentration (e.g. through generation of a standard curve). Relative quantification can be accomplished, for example, by comparison of signals between two or more target nucleic acids.

The method and diagnostic kit of the invention relate to the amplification, detection and quantification of one or more target nucleic acids in a sample, wherein the amplification of the target nucleic acid(s) is monitored preferably in real-time. The method according to the invention may be used for detecting in a sample the presence or absence of one or more target nucleic acids. It may also be used for quantifying the amount/concentration of one or more target nucleic acids in a sample.

The method of the invention advantageously allows for the detection of a multitude of target nucleic acids of interest in one reaction (multiplexing) using only detectable labels of a single color. The method disclosed herein further provides the advantage of being highly time- and cost-efficient and furthermore provides an improved signal-to-background ratio over the methods comprised in the prior art.

In a first aspect, the invention relates to a method for monitoring the amplification of one or more target nucleic acids comprising the following steps:
a) providing a substrate having immobilized upon its surface a multitude of oligonucleotide primers;
b) adding to said substrate a sample of one or more target nucleic acids and further reagents required for nucleic acid amplification and labeling in a polymerase chain reaction process;
c) amplifying the one or more target nucleic acids by a process involving thermocycling, comprising the steps of:
   i) annealing the one or more target nucleic acids to corresponding oligonucleotide primers immobilized on the surface; and
   ii) extension of the immobilized oligonucleotide primers, wherein the immobilized oligonucleotide primers get detectably labeled during extension;
d) measuring the signal of the extended and labeled oligonucleotide primers during or after at least one thermal cycle.

By "monitoring the amplification" is meant that the presence, absence or quantity of one or more target nucleic acids is determined using the method of the invention.

Substrates used for the invention can be of any geometric shape. The substrate may e.g. be planar or spherical (e.g. a bead). It may e.g. be in the form of particles, strands, sheets, tubing, spheres, containers, capillaries, plates, microcopy-slides, beads, membranes, filters etc. In a preferred embodiment the substrate is planar and solid. In this context, solid means that the substrate is substantially incompressible. Suitable materials for the substrate include e.g. glass, plastic, nylon, silica, metal or polymers. In some embodiments the substrate might be magnetic.

In a preferred embodiment polymer and/or glass surfaces are used. Suitable polymers for the substrate are e.g. cyclic olefin polymer (COP) or cyclic olefin copolymer (COC).

Preferably, the substrate is thermally stable (e.g. up to 100°C) such that it is able to endure the temperature conditions typically used in PCR. It is further preferred that the substrate is capable of being modified by the attachment of oligonucleotide primers. The oligonucleotide primers are preferably immobilized on the surface of the substrate by covalent attachment.

Oligonucleotide primers and/or the surface of the substrate may be modified with functional groups such as e.g. hydroxyl, carboxyl, phosphate, aldehyde or amino groups.

In some embodiments a chemical linker, linking the primers and the substrate, may be used for covalently attaching the oligonucleotide primers to the substrate. For example, a thymine tail may be used as a linker in order to attach the oligonucleotide primers to the substrate. A thymine tail may e.g. comprise about 2, 4, 6, 8, 10, 12, 14, 16, 18, 20 or more than 20 thymines. In a preferred embodiment a thymine linker comprises 16 thymines. In some preferred embodiments the linker may further comprise abasic sites located between the thymine tail and the oligonucleotide primer. For example, the linker may comprise 1 to 20 abasic sites.

In general any nucleotide linker or any other suitable linker known to the skilled person can be used. In cases where a linker is used, the linker is preferably attached to the 5' end of the oligonucleotide primer.

Oligonucleotide primers may alternatively be adsorbed on the surface of the substrate, provided that they remain stably attached to the surface under thermocycling conditions.

Oligonucleotide primers can be single stranded oligonucleotide molecules, for example single stranded DNA or RNA molecules.

Preferably, the oligonucleotide primers immobilized upon the substrate in step
a) have a length of about 5 to 100 nucleotides, preferably of about 10 to 50 nucleotides, most preferably of about 15 to 30 nucleotides. In a further preferred embodiment, the oligonucleotide primers may have a length of about 5 to 10, 10 to 15, 15 to 20, 10 to 20, 20, 21, 22, 23, 24, 25, 20 to 25, 25 to 30, 10 to 30, 10 to 20, 30 to 35, 35 to 40, 10 to 40, 20 to 40, 30 to 40, 40 to 45, 45 to 50, 20 to 50, 30 to 50, 40 to 50, 50 to 55, 55 to 60, 60 to 65, 65 to 70, 60 to 70, 70 to 75, 75 to 80, 80 to 85, 85 to 90, 70 to 90, 90 to 95 or 95 to 100 nucleotides.

If the method according to the invention is used to monitor the amplification of a single target nucleic acid, all oligonucleotide primers immobilized upon the surface of the substrate may be specific for the same target nucleic acid. However, the method according to the invention is preferably used for the detection of a plurality of different target nucleic acids. Thus, in a preferred embodiment the multitude of oligonucleotide primers are capable of specifically binding to a plurality of different target nucleic acids.

In some embodiments, each individual oligonucleotide primer immobilized upon the substrate may be specific for only one target nucleic acid. Alternatively, individual oligonucleotide primers immobilized upon the substrate may be specific for more than one target nucleic acid.

For example, individual primers may be specific for various homologous sequences. A given oligonucleotide primer may for example be specific for 1, 2, 3, 4, 5, 6, 7, 8, 9, 10, more than 10, more than 20, more than 30, more than 40 or more than 50 target nucleic acids. If a given oligonucleotide primer is specific for more than 1 (i.e. several) target nucleic acids then it is preferred as described below that these target nucleic acids are similar.

If an oligonucleotide primer is specifically binding a target nucleic acid, such as e.g. a gene, cDNA or RNA, then it is preferred that the oligonucleotide primer specifically binds to the 5'- or 3'-end of an open reading frame of said target nucleic acid.

An open reading frame (ORF) is a portion of an mRNA, cDNA or a gene which is located between and includes the start codon (also called initiation codon) and the stop codon (also designated as termination codon) of said mRNA, cDNA or gene. One ORF typically encodes one protein. Thus, an ORF is part of the sequence that will be translated by the ribosomes into the corresponding protein.

In a preferred embodiment an oligonucleotide primer comprises the initiation codon at the 5'-end or the termination codon at the 3'-end of the mRNA, cDNA or of the gene but not both at the same time. In another further preferred embodiment, the oligonucleotide primers specifically bind to the 5' or 3' end of an open reading frame and have a length of about 15 to 30 nucleotides.

In a further preferred embodiment control oligonucleotides having the same nucleotide sequence as the oligonucleotide primers immobilized on the surface of the substrate are tested in an independent nucleic acid amplification experiment for their capability to hybridize to a target nucleic acid of interest. Preferably, such an experiment is performed at the same experimental conditions as the method according to the invention and is performed prior to performing the method according to the invention. Such a control experiment is particularly preferred in cases where the method according to the invention is used to determine the presence of absence of one or more target nucleic acid(s) in a sample.

For multiplexing it may be preferable to pattern the surface of the substrate, i.e. to locate immobilized oligonucleotide primers to different regions on the substrate. Thus, in a preferred embodiment of the method of the invention the multitude of oligonucleotide primers can be located to separate regions on the surface of the substrate. As used herein, "separate regions" refer to non-overlapping regions on the surface of the substrate. "Separate regions" can contact each other or can be arranged on the surface of the substrate such that they do not contact each other.

Preferably, separate regions are independently addressable regions, also referred to as "spots". In a preferred embodiment, a spot comprises at least 1, 2, 3, 4, 5, 10, 20, 30, 40, 50, 60, 70, 80, 90, 100, 200, 300, 400, 500, 600, 700, 800, 900, 1000, 5000, 10000, 100000 or at least 1000000 oligonucleotide primers. It is not required that each spot comprises the exact same number of oligonucleotide primers but it is preferred that all spots comprise a similar number of oligonucleotide primers such that upon measuring, the signal of all spots can be compared with each other. For example, the surface of the substrate may comprise multiple spots each of which consists of a sufficient number of oligonuclotide primers that can be detected in step (d) of the method of the invention.

In some embodiments the solid substrate may comprise about 1, 2, 3, 4, 5, 6, 7-10, 10-50, 50-100, 100-500, 500-1,000, 1,000-5,000, 5,000-10,000, 10,000-50,000, 50,000-100,000, 100,000-500,000, 500,000-1,000,000 or more than 1,000,000 spots.

In one embodiment, the substrate may comprise between 4 and 100000 spots per cm² and preferably between 20 and 1000 spots per cm².

Preferably, spots have a diameter of 50 to 250 µm. In further preferred embodiments spots may have a diameter of 50 to 90 µm, 90 to 120 µm, 120 to 150 µm, 150 to 180 µm, 180 to 200 µm, 200 to 220 µm or 220 to 250 µm.

It is further preferred that spots have a pitch of 100 to 500 µm. Spots may also have a pitch of 100 to 200 µm, 200 to 300 µm, 300 to 400 µm or 400 to 500 µm.

In a particular preferred embodiment spots have a diameter of 50 to 250 µm and a pitch of 100 to 500 µm. Most preferably, spots have a diameter of about 200 µm and a pitch of about 400 µm.

It is further preferred that in the method according to the invention the oligonucleotide primers within each individual region are capable of specifically binding to the same or similar target nucleic acids. Two target nucleic acids are "similar" if they share at least 70%, 80%, 85%, 90%, 95%, 96%, 97%, 98%, 99% or 99,5% sequence identity. The determination of percent identity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. MoI. Biol. 215: 403-410 available at NCBI (http://www.ncbi.nlm.nih.gov/blast/Blast.cge). The determination of percent identity is performed with the standard parameters of the BLASTn and BLASTp programs. If determining the percent identity between two sequences then it is preferred that the percent sequence identity is determined over the entire length of the shorter of the two sequences only.

In another preferred embodiment of the method, some or all regions on the substrate differ from each other in that their oligonucleotide primers are capable of specifically binding to different target nucleic acids. Preferably 10%, 20%,30%, 40%, 50%, 60%,70%, 80%, 90%, or 100% of the regions on the substrate differ from each other in that their oligonucleotide primers are capable of specifically binding to different target nucleic acids.

In a preferred embodiment the oligonucleotide primers have their 5'- terminal ends attached to the substrate, such that their 3'-terminal ends are free to participate in primer extension reactions. The oligonucleotide primers can be synthesized directly on the substrate or can be attached to the substrate postsynthetically. The primers may be deposited on the surface of the substrate e.g. by spotting or any other method comprised in the art known by the average skilled person. Thus, advantageously the manufacturing of the substrate requires no difficult, expensive or time consuming manufacturing steps but merely involves attaching the oligonucleotide primers to the substrate. Furthermore manufactured substrates comprising said immobilized oligonucleotide primers can easily be stored and exhibit a long shelf life. Furthermore, the manufactured substrate can be stored under dry conditions.

In step b) of the method of the invention, one or more target nucleic acids and further reagents required for nucleic acid amplification and labeling in a polymerase chain reaction process are added to the substrate. This serves to set up a nucleic acid amplification reaction.

The sample of one or more target nucleic acids according to b) may comprise one or more target nucleic acids to be detected or measured by the method of the invention.

In a preferred embodiment, the one or more target nucleic acid(s) in step b) comprise deoxyribonucleic acid(s) and/or ribonucleic acid(s).

If the nucleic acid sample according to b) comprises more than one target nucleic acids, the target nucleic acids may be derived from the same origin or from different origins, for example different biological specimens. The more than one target nucleic acids will typically comprise nucleic acids having different sequences. In a preferred embodiment the sample of one or more target nucleic acid(s) comprises nucleic acids whose sequences are complementary to one or more of the oligonucleotide primers immobilized on the substrate. It is however not required that all target nucleic acids comprised in the sample are capable of binding to the oligonucleotide primers comprised on the substrate. For example, the method according to the invention may in some embodiments be used for determining the presence or absence of a specific target nucleic acid in a sample. In such cases a query sample of one or more possible target nucleic acids may be added to the substrate in step b) in order to determine whether the sample comprises one or more nucleic acid targets of interest. If such nucleic acid targets are present in the sample, they will be capable of binding to the oligonucleotide primers on the surface of the substrate. If however the query sample does not comprise any of the nucleic acid targets of interest or if the sample comprises a mixture of target nucleic acid(s) in question and additional nucleic acids of no particular interest, none or only some of the nucleic acids in the sample will bind to the oligonucleotide primers comprised on the substrate.

One advantage of the method of the invention is that it is capable of multiplex analysis. For example, the sample of target nucleic acids may comprise 1, 2, 3, 4, 5, 6, 7, 8, 9, 10, more than 10, more than 20, more than 30, more than 40, more than 50, more than 60, more than 70, more than 80, more than 90, more than 100, more than 150, more than 200, more than 300, more than 400, more than 500, more than 1000, more than 5000, more than 10,000, more than 100,000 or more than 1,000,000 different target nucleic acids.

The one or more target nucleic acids may be of eukaryotic bacterial or viral origin.

In addition to the one or more target nucleic acids further reagents required for nucleic acid amplification and labeling in a polymerase chain reaction process are added to the substrate. It is known to the skilled person, which reagents need to be added to a nucleic acid target in order to amplify said nucleic acid target.

Preferably, the reagents required for nucleic acid amplification and/or labeling in b) are provided in solution, preferably in the form of a reaction mixture. It is further preferred that the substrate is in contact with the solution during amplification.

In a further preferred embodiment the reagents required for nucleic acid amplification and labeling comprise an oligonucleotide primer pair or a plurality of different oligonucleotide primer pairs, nucleotides, at least one polymerase and a detectable label. The reagents for nucleic acid amplification and labeling may further comprise a reaction buffer.

In a preferred embodiment, the detectable label is a fluorescently labeled nucleotide. "Nucleotides" may be ribonucleotides or deoxyribonucleotides. Preferably, nucleotides are deoxyribonucleotides. If fluorescently labeled nucleotides are comprised in the reagents then they will be incorporated into the extended immobilized oligonucleotide primers during thermocycling. Thus, in a preferred embodiment, the immobilized oligonucleotide primers get labeled with a fluorescent label during the extension in step c). This implies that the immobilized oligonucleotide primers are not fluorescent in step a).

Suitable fluorescent labels may comprise e.g. Cyanine dyes, such as e.g. Cyanine 3, Cyanine 5 or Cyanine 7, Alexa Fluor dyes, such as e.g. Alexa 594, Alexa 488 or Alexa 532, fluorescein family dyes, Texas Red and Rhodamine. In some embodiments the nucleotides may be labeled with two or more different dyes. In a preferred embodiment the nucleotides are labeled with only one dye.

When using fluorescently labeled nucleotides a mixture of labeled and unlabelled nucleotides may be used. In one embodiment the unlabeled nucleotides are used in excess amount, i.e. in an amount which is greater than the amount of the fluorescently labeled nucleotides. Preferably at least three or four fold more unlabeled nucleotides are used than fluorescently labeled nucleotides.

If during thermocycling (see below) melting curves need to be recorded, the reagents required for nucleic acid amplification and labeling may additionally comprise a nucleic acid stain. A nucleic acid stain, for example SYBR Green, is an intercalating fluorophore which can be used to determine the amount of double stranded DNA. Such intercalating fluorophores have the property that their fluorescence is enhanced when they are intercalated into DNA than when in solution. Thus, the amount of melted (single stranded) versus non-melted (double stranded) DNA can be determined as is known in the art. If melting curves are recorded, then the fluorescent dye has preferably a different excitation wavelength than the fluorescent nucleotides that may also be used in the method.

As mentioned above, the reagents required for nucleic acid amplification and labeling may comprise an oligonucleotide primer pair or a plurality of different oligonucleotide primer pairs. In cases where the reagents required for nucleic acid amplification and labeling comprise a plurality of different primer pairs, the different primer pairs are preferably specific for different target nucleic acids. The reagents might e.g. comprise 1, 2, 3, 4, 5, 6, 7, 8, 9, 10, more than 10, more than 20, more than 30, more than 40, more than 50, more than 60, more than 70, more than 80, more than 90, more than 100, more than 150, more than 200, more than 300, more than 400, more than 500, more than 1000, more than 5000, more than 10,000, more than 100,000 or more than 1,000,000 primer pairs.

The reagents required for nucleic acid amplification and labeling may comprise forward and/or reverse primers specific for said one or more target nucleic acids. If forward and reverse primers are comprised in the reagents, this will allow for a start-up reaction to occur in solution, generating amplicon(s).

The complementary strands of the amplicons are capable of annealing to the oligonucleotide primers immobilized on the substrate during thermocycling in step c) and thus provide additional copies of the target nucleic acids available for extension and labeling of said oligonucleotide primers. Preferably, either the forward or the reverse primer present in the reagents required for nucleic acid amplification and labeling has the same sequence as the oligonucleotide primers immobilized on the substrate.

In some embodiments the reagents required for nucleic acid amplification and labeling may comprise only the forward primer or the reverse primer of each primer pair. In these cases it is preferred that the second primer of each primer pair is immobilized upon the surface of the substrate. Under these circumstances amplicon(s) will not be generated within the reaction solution, i.e. only extension of oligonucleotide primers on the surface of the substrate will occur.

The terms "forward primer" and "reverse" primer are used herein according to their conventional and well known meaning in the art.

The reagents required for nucleic acid amplification and labeling may further comprise a DNA polymerase. In some embodiments the reagents may in addition to the DNA polymerase comprise a reverse transcriptase. A reverse transcriptase is preferably added when the one or more nucleic acid probe(s) in step b) comprises messenger RNA. For further details see below.

Amplification of the one or more target nucleic acids from step b) is achieved by a process involving thermocycling. The term thermocycling as used herein refers to a process comprising alternating heating and cooling of a reaction mixture allowing for amplification of one or more target nucleic acids. Alternating heating and cooling is repeated in several cycles, herein referred to as thermal cycles.

In some embodiments a thermal cycle may e.g. comprise 3 different temperature steps, which may e,g. comprise alternating from a first temperature step of from about 90°C to about 100°C (denaturation), to a second temperature step of from about 40°C to about 75°C (annealing), preferably from about 50°C to about 70°C to a third temperature step of from about 70° C to 80° C, preferably about 72°C to 75° C (extension). Alternative forms of thermocycling are well known in the an and may comprise less or different temperature steps.

Preferably, thermocycling in step c) comprises more than 5 thermal cycles, more than 10 thermal cycles, more than 20 thermal cycles, more than 30 thermal cycles or more than 50 thermal cycles. Most preferably thermocycling in step c) comprises about 5 to 50 thermal cycles.

In a preferred embodiment the process involving thermocycling is polymerase chain reaction (PCR). PCR in addition to the aforementioned thermal cycles may further comprise a single initialization step comprising heating the reaction mixture to about 92° C to 100 ° C and/or a cooling step at about 4° C after thermocycling has completed.

According to the method disclosed herein, amplifying the one or more target nucleic acids by a process involving thermocycling, comprising the steps of:
i) annealing the one or more target nucleic acids to corresponding oligonucleotide primers immobilized on the surface; and
ii) extension of the immobilized oligonucleotide primers, wherein the immobilized oligonucleotide primers get detectably labeled during extension.

In some embodiments annealing of the one or more target nucleic acids to corresponding oligonucleotide primers may e.g. be achieve in a temperature step of from about 40°C to about 75°C and extension of the immobilized oligonucleotide primers may e.g. be achieved in a temperature step of from about 70°C to 80°C, preferably about 72° C to 75° C as described above, In other embodiments annealing may take place at the same temperature as the extension.

In step c) i) in addition to target nucleic acids provided in step b) also complementary strands of already generated amplicons may anneal to their corresponding oligonucleotide primers.

Thermocycling is usually performed in a suitable apparatus, i.e. a thermal cycler.

In some embodiments it might be desirable to perform gene expression analysis, i.e. to determine the transcription levels of one or several different genes. In such cases a sample comprising mRNA transcript(s) of one or several genes of interest may be provided. The mRNA transcript(s) may then be reversed transcribed into cDNA. In some embodiments reverse transcription may be performed prior to the above thermocycling reaction in a separate reverse transcriptase PCR reaction. cDNA resulting from such a reaction may then serve as a target nucleic acid added to the substrate in step b).

In a preferred embodiment cDNA synthesis and the amplification step in c) can be performed within the same reaction mixture. In these cases it is preferred that the one or more target nucleic acid(s) in step b) comprise messenger RNA and the further reagents required for nucleic acid amplification and labeling in addition to a DNA polymerase, such as e.g. Taq polymerase, comprise a reverse transcriptase.

If such an approach is used, it is preferred that the amplification process in c) prior to the above described thermocycling comprises a single incubation step performed at about 40° C to 60 °C allowing for cDNA synthesis. In one embodiment such an incubation step may be performed within 10 min to 45 min. In some embodiments the reaction may be incubated at about 20° C to 25° C prior to the cDNA synthesis step. Such an incubation may e.g. be performed within 5 to 20 minutes.

In step d) of the method of the invention the signal of the extended and labeled oligonucleotide primers is measured. The measurement may be taken during or after at least one thermal cycle. Preferably, the signal of the extended labeled oligonucleotide primers is measured during or after at least 5 thermal cycles, during or after at least 10 thermal cycles, during or after least 15 thermal cycles, during or after at least 20 thermal cycles during or after at least 25 thermal cycles. Most preferably the fluorescent signal of the extended labeled oligonucleotide primers is measured during or after every thermal cycle.

The increase in the amount of nucleic acid is thus monitored as it is amplified, i.e. the increase in the amount of nucleic acid is measured in "real time".

"Real time measurement" may include detection of the kinetic production of signal, comprising taking a plurality of measurements in order to characterize the signal over a period of time. The fluorescence intensity for each amplification reaction may be determined using, e.g., a charge-coupled device (i.e. CCD camera or detector) or other suitable instrument capable of detecting the emission spectra for the label molecules used.

One problem that frequently occurs with signal detection is that it is often hampered by high background fluorescence. For example, high background fluorescence may be caused by unbound fluorescently labeled molecules such as, e.g., labeled nucleotides and/or labeled amplicons that are present in the reaction solution during thermocycling. This may render the measurement less sensitive.

Thus, in the context of the present invention it is desirable to only measure the signal of the extended labeled primers near the surface of the substrate.

Therefore, in one preferred embodiment a highly sensitive surface specific detection technology is used. Such a technology allows to limit the measurement to a small volume nearby the surface of the substrate and to detect labeled molecules on the surface of the substrate while largely avoiding detection of labeled molecules in solution, thus providing an improved signal-to-background ratio.

In one preferred embodiment detection of the signal is achieved by use of a confocal fluorescence scanner or an evanescence wave microcopy technology. An evanescent wave is a nearfield standing wave exhibiting exponential decay with distance. For example, a total internal reflection fluorescence (TIRF) microscope can be used to measure the signal. In another preferred embodiment detection of the signal is achieved by use of a luminescence sensor. Such a device is e.g. described in WO 2007/010428, which is herewith incorporated by reference. Alternatively, the signal may e.g. be detected by use of a confocal microscope.

Thus, in a preferred embodiment, in step d) only labels are measured which are located within a distance of about 100 nm to about 300 nm, preferably within 100 nm to 200 nm and most preferably within 100 nm to 150 nm from the surface of the substrate when using an evanescent detection scheme or wherein in step d) only labels are measured which are located within a distance of about 1 µm or less when using a confocal detection scheme.

For each amplification reaction, the measured emission spectra obtained from the fluorescence samplings form an amplification data set. In some embodiments, it might be desirable to detect the signal for each of the primer extension amplification reactions in order to determine the presence or absence of one or more target nucleic acids in the sample, wherein the absence of the respective signal correlates with the absence of the respective target nucleic acid.

In other embodiments the amplification data set that may be processed for quantification, i.e. to determine the initial concentration of the one or more target nucleic acid(s). In some embodiments, the amplification data set may further comprise fluorescence intensity data obtained from one or more control nucleic acid targets whose initial target concentration is known, such as for example mRNA encoding the enzyme GAPDH. It is then possible to compare the signal intensity of target nucleic acids of unknown concentration with the target nucleic acids of known concentration, e.g. through generation of a standard curve.

A computer may be used for data collection and processing. Data processing may e.g. be achieved by using a suitable imaging software.

For further details on real-time PCR methodology and signal detection and quantification, reference can e.g. also be made to Dorak, M. Tevfik (ed.), Real-Time PCR, April 2006, Taylor & Francis; Routledge, 978-0-415-37734-8.

The method disclosed herein provides the advantage that the amplification reaction is performed directly on the surface of the substrate by incorporation of fluorescently labeled nucleotides during extension of the immobilized oligonucleotide primers. It is thus not necessary to hybridize any soluble labeled amplification products to the surface of the substrate in an additional hybridization step for detection, as frequently performed in the prior art. The method of the present invention is, thus more efficient and less time consuming than methods requiring an additional hybridization step (requiring an additional temperature step).

If an evanescent wave detection method is used the excitation wave will exhibit an exponential decay and, thus fluorescent labels which are further remote from the surface of the substrate will have a reduced emission signal. Thus, when using shorter oligonucleotide primers in the preferred embodiments of the method of the invention, labeled nucleotides will be incorporated and detected closer to the surface. Alternatively, confocal (diffraction limited) detection can be used where only labels are measured which are located within a distance of about 1 µm or less from the surface of the substrate.

Furthermore, the incorporation of detectably labeled nucleotides into the oligonucleotide primers immobilized on the substrate provides the advantage of a permanent signal which is present near the surface during the entire amplification procedure. The signal can even be detected at higher temperatures (e.g. at a temperature higher than 90°C). For example, the signal can even be detected after denaturation. This is particularly advantageous as the inventors of the present invention have surprisingly found that if the detection of the signal is performed at high temperatures, e.g. at the denaturation temperature, an improved signal-to-background ratio is achieved.

Additionally, incorporation of multiple detectably labeled nucleotides into the olignucleotide primers immobilized on the substrate results in an amplification of the detectable signal. This provides the further advantage that in comparison to other real-time amplification methods comprised in the art, a lower number of thermal cycles will be required until sufficient label is present to reach a detectable signal, thus allowing for faster analysis assays based on the method of the invention.

The fluorescent signal originating from the extended labeled olignucleotide primers can be enhanced by increasing the concentration of the detectably labeled nucleotides present in the reagents required for nucleic acid amplification and labeling.

The invention in a further aspect relates to the use of a method according to the invention for detecting in a sample the presence, absence or the amount of one or more target nucleic acids.

A "sample" may for example be a material or mixture of materials, typically, although not necessarily, in fluid form, containing one or more target nucleic acids of interest. Samples may e.g. be biological samples obtained from natural biological sources, such as cells or tissues. The samples may e.g. also be derived from tissue biopsies and other clinical procedures. Examples of samples are also blood samples, forensic samples, food samples, or environmental samples, such as e.g. water samples.

In another aspect the present invention also relates to a diagnostic kit for monitoring the amplification of one or more target nucleic acids, comprising:
(i) a substrate having immobilized upon its surface a multitude of oligonucleotide primers; and
(ii) one or more target nucleic acids and further reagents required for nucleic acid amplification and labeling in a polymerase chain reaction process;
wherein the reagents required for nucleic acid amplification and labeling comprise nucleotides labeled with a fluorescent dye.

The substrate having immobilized upon its surface a multitude of oligonucleotide primers and the reagents required for nucleic acid amplification and labeling in a polymerase chain reaction process have been described above.

The contents of the kit can be used to carry out the method of the invention. In some embodiments the kit may also comprise one or more target nucleic acids such as for example internal control template target nucleic acids.

The aforementioned components may be packaged in containers such as e.g. vials, test tubes, flasks, bottles, syringes or other containers into which a component may be placed. Preferably the components are suitably aliquoted. The kits typically also comprise packaging for containing the various components for commercial sale. A kit may also include instructions for employing the kit components.

In general, any of the components disclosed herein may be combined in a kit.

### Examples

A hybridization experiment was performed where hybridization of an HPV 16 fragment on an array was monitored.

The array was obtained by printing spots with capture probes (among which spots specific for HPV 16) on an amino-functionalized microscope slide (SuperAmine slides, available from Arraylt, www.arrayit.com).

Then a PCR amplicon of HPV16 was obtained using fluorescently labeled primers. The amplified amplicon thus contained fluorescently labeled nucleotides.

The diluted PCR product, about 60 pM HPV-16 fluorescently amplicons and 20 nM fluorescently labeled primers, were hybridized on the array and hybridization over temperature was monitored by scanning the array in intervals of 1 minute using a home-made confocal scanner. The background was determined by taking the average fluorescent signal in a region next to the spot with HPV 16 capture probes. The hybridization buffer consisted of PCR buffer (1x GeneAmp, Applied Biosystems).

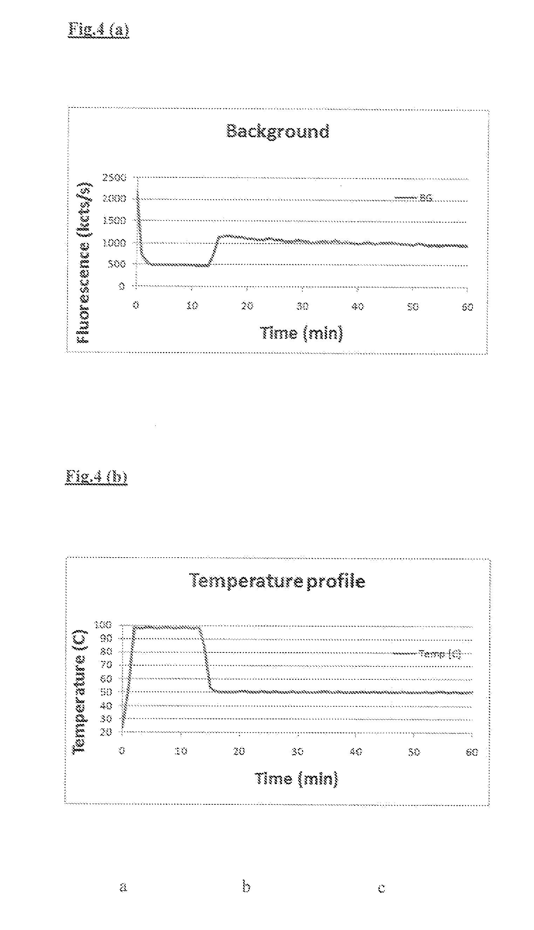

Figure 4(a) shows fluorescent background signal as a function of time. The experiment was started at room temperature, the temperature was then raised to the denaturation temperature and hybridization was finally performed at an intermediate temperature of 50° C. Temperature of heating electrode (temperature in hybridization chamber is a few degrees lower) as a function of time.

Fig.5(b) shows that the fluorescent background decreases with increasing temperature. During the denaturation step (electrode temperature of 98° C), the background is substantially lower than during the hybridization step (electrode temperature of 50 C) and at room temperature.

The presence of a fluorescent background limits the contrast (between the spots with capture probes and the background) in the measurements. It is therefore preferable to perform the hybridization at an elevated temperature, such as during the denaturation step. However, during the denaturation step also the HPV 16 amplicons specifically hybridized to the spots with capture probes are denatured from the capture sites, so there is essentially no detectable hybridization at these temperatures.

If primers being immobilized on the surface of an array are used together with e.g. labeled nucleotides in the PCT reaction, the immobilized primers will become permanently labeled. The detection can then be performed at high temperatures during each cycle.

## Claims

1. A method for monitoring the amplification of one or more target nucleic acids comprising the following steps:
a) providing a substrate having immobilized upon its surface a multitude of oligonucleotide primers;
b) adding to said substrate a sample of one or more target nucleic acids and further reagents required for nucleic acid amplification and labeling in a polymerase chain reaction process;
c) amplifying the one or more target nucleic acids by a process involving thermocycling, comprising the steps of:
i) annealing the one or more target nucleic acids to corresponding oligonucleotide primers immobilized on the surface; and
ii) extension of the immobilized oligonucleotide primers, wherein the immobilized oligonucleotide primers get detectably labeled during extension;
d) measuring the signal of the extended and labeled oligonucleotide primers during or after at least one thermal cycle.

2. The method according to claims 1, wherein the multitude of oligonucleotide primers are capable of specifically binding to a plurality of different target nucleic acids.

3. The method according to claim 1 or 2, wherein the multitude of oligonucleotide primers are located to separate regions on the surface of the substrate.

4. The method according to claim 3, wherein the oligonucleotide primers within each individual region are capable of specifically binding to the same or similar target nucleic acids.

5. The method according to claim 3 or 4, wherein some or all regions on the substrate differ from each other in that their oligonucleotide primers are capable of specifically binding to different target nucleic acids.

6. The method according to any of claims 1 to 5, wherein the immobilized oligonucleotide primers get labeled with a fluorescent label during the extension in step c).

7. The method according to claim 6, wherein the fluorescent label is a fluorescently labeled nucleotide.

8. The method according to any of the preceding claims, wherein the oligonucleotide primers immobilized upon the substrate in step a) have a length of about 5 to 100 nucleotides, preferably of about 10 to 50 nucleotides, most preferably of about 15 to 30 nucleotides.

9. The method according to any of the preceding claims, wherein the oligonucleotide primers specifically bind to the 5' or 3' end of an open reading frame of a target nucleic acid.

10. The method according to any of claims 1 to 9, wherein in step d) only labels are measured which are located within a distance of about 100 nm to about 300 nm from the surface of the substrate when using an evanescent detection scheme or wherein in step d) only labels are measured which are located within a distance of about1 µm or less using a confocal detection scheme.

11. The method according to any of the preceding claims, wherein the fluorescent signal of the extended labeled oligonucleotide primers is measured in step d) during or after at least 2 thermal cycles, during or after at least 5 thermal cycles, during or after at least 10 thermal cycles, during or after least 15 thermal cycles, during or after at least 20 thermal cycles or during or after at least 25 thermal cycles.

12. The method according to any of claims 1 to 11, wherein the reagents required for nucleic acid amplification and labeling comprise forward and/or reverse primers specific for said one or more target nucleic acids.

13. The method according to any of claims 1 to 12, wherein the thermocycling in c) comprises about 5 to 50 thermocyles

14. Use of a method according to any of claims 1 to 13 for detecting in a sample the presence, absence or the amount of one or more target nucleic acids.

15. A diagnostic kit for monitoring the amplification of one or more target nucleic acids, comprising:
(i) a substrate having immobilized upon its surface a multitude of oligonucleotide primers; and
(ii) further reagents required for nucleic acid amplification and labeling in a polymerase chain reaction process;
wherein the reagents required for nucleic acid amplification and labeling comprise nucleotides labeled with a fluorescent dye.
